# EUROPÄISCHE PATENTSCHRIFT

(11) **EP 2 180 908 B1**
(45) Veröffentlichungstag und Bekanntmachung des Hinweises auf die Patenterteilung: **15.02.2012**
(21) Anmeldenummer: 08758044.5
(22) Anmeldetag: 09.05.2008
(51) Int. Cl.: A61M 1/16, A61L 2/04, C02F 1/02

(54) **ANORDNUNG ZUR WASSERVERSORGUNG EINES DIALYSEGERÄTS**
ARRANGEMENT FOR SUPPLYING WATER TO A DIALYSIS DEVICE
DISPOSITIF D'ALIMENTATION EN EAU D'UN APPAREIL DE DIALYSE

(30) Priorität: 15.05.2007 DE 102007022547
(43) Veröffentlichungstag der Anmeldung: 05.05.2010
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen
(86) Internationale Anmeldenummer: PCT/DE2008/000786
(87) Internationale Veröffentlichungsnummer: WO 2008/138311

(56) Entgegenhaltungen:
- DE-A1- 10 013 964
- DE-A1- 10 111 104
- DE-A1- 19 933 223
- US-A1- 2003 034 305

## Beschreibung

Die Erfindung betrifft eine Anordnung zur Wasserversorgung eines Dialysegerätes, mit einer Verteilerleitung einer zentralen Wasseraufbereitungseinrichtung und einem Dialysegerät, das an die Verteilerleitung angekoppelt ist.

Beim Einsatz von Dialysegeräten in Krankenhäusern werden häufig mehrere dieser Geräte räumlich und funktionell zusammengefasst, so dass bestimmte Funktionen zentralisiert werden können. Hierzu zählt insbesondere die gemeinsame Versorgung mit aufbereitetem Wasser über ein Verteilungssystem, gewöhnlich in Form einer Ringleitung.

An die Qualität des den Dialysegeräten zugeführten Wassers werden erhebliche Anforderungen gestellt, u.a. hinsichtlich der Abwesenheit von Mikroorganismen und deren Stoffwechsel- und Zerfallsprodukten. Die eingesetzten Wasseraufbereitungseinrichtungen, insbesondere Umkehrosmoseanlagen, sind zwar aufgrund ihres Funktionsprinzips geeignet, diesen Anforderungen zu entsprechen, jedoch ist im praktischen Betrieb das Eindringen von Mikroorganismen nicht völlig auszuschließen. Daher ist es üblich, in geeigneten Zeitabständen eine Desinfektion durchzuführen. Zur Anwendung kommen Verfahren der chemischen, thermischen oder kombiniert chemisch-thermischen Desinfektion. Nach einer chemischen Desinfektion ist in jedem Falle eine ausreichende Spülung zur Beseitigung von Desinfektionsmittel-Rückständen notwendig.

Die Desinfektion einer Hämodialyseanlage, die aus einem zentralen Wasseraufbereitungssystem und einer Verteilerleitung (Ringleitung) mit mehreren angeschlossenen Dialysegeräten besteht, kann in der Weise erfolgen, dass die Desinfektionslösung über das gesamte System verteilt wird und dabei die angeschlossenen Dialysegeräte in die Desinfektion einbezogen werden. Dabei erstreckt sich die Desinfektion und nachfolgende Spülung auch auf die Anschlussleitungen dieser Geräte.

Es besteht jedoch häufig auch das Bedürfnis, Dialysegeräte einzeln einer chemischen Desinfektion zu unterziehen. Dies ist dadurch gerechtfertigt, dass in dem flüssigkeitsführenden System des Dialysegeräts relativ günstige Vermehrungsbedingungen für Mikroorganismen vorliegen, bedingt durch die Temperatur und Zusammensetzung der im Gerät erzeugten Dialysierflüssigkeit, die annähernd physiologischen Verhältnissen entspricht. Zum Zwecke der Desinfektion wird mittels entsprechender Einrichtungen des Dialysegeräts die Desinfektionslösung aus zugeführtem Wasser und Desinfektionsmittel-Konzentrat im Dialysegerät selbst zubereitet und zirkuliert. Nach dem Stand der Technik kann bei diesem Verfahren jedoch die Anschlussleitung, die das Dialysegerät mit der Verteilerleitung des zentralen Wasserversorgungssystems verbindet, nicht in die Desinfektion einbezogen werden. Damit besteht das Risiko, dass sich Mikroorganismen, die in diesem Bereich angesiedelt sind - z.B. an den Leitungsinnenflächen (sog. Biofilm) - in relativ kurzer Zeit erneut ausbreiten.

Die DE 10 2005 031 334 A1 befasst sich damit, die Anschlussleitung, die ein Dialysegerät mit der Verteilerleitung verbindet, in die Desinfektion dieses einzelnen Dialysegerätes ein zu beziehen. Hierzu ist ein Adapter vorgesehen, der im wesentlichen aus zwei Leitungen besteht, die mittels eines Verzweigungsteils von einem Zuleitungsabschnitt abzweigen, der mit der Verteilerleitung verbunden ist. Bei einem Desinfektionsvorgang durchströmt die Desinfektionsflüssigkeit die beiden Leitungsabschnitte, die mit dem Leitungssystem des Dialysegerätes verbunden sind. Dabei sind zwei Absperrventile in dem Zuleitungsabschnitt angeordnet, um zu verhindern, dass Desinfektionsflüssigkeit in die Verteilerleitung gelangt.

Die DE 199 33 223 A1 offenbart eine Anordnung zur Wasserversorgung eines Dialysegeräts, wobei ein Zulaufgefäß in einem Dialysegerät angeordnet ist.

Der Erfindung lag die Aufgabe zugrunde, den Anschluss eines Dialysegeräts an die Verteilerleitung so zu gestalten, dass die Anschlussleitung, die das Dialysegerät mit der Verteilerleitung verbindet, in die Desinfektion des einzelnen Dialysegeräts einbezogen wird, wobei verhindert sein soll, dass Desinfektionsflüssigkeit in die Verteilerleitung gelangen kann.

Diese Aufgabe wird durch die Merkmale des Patentanspruchs 1 gelöst.

Vorteilhafte Ausgestaltungen der Erfindung sind in den Unteransprüchen gekennzeichnet.

Die vorliegende Erfindung sieht andere Mittel als die DE 10 2005 031 334 A1 vor, um auszuschließen, dass die Verteilerleitung durch Desinfektionsflüssigkeit verunreinigt wird. Diese Mittel enthalten eine Flusskammer, die mit ihrem oberen Teil über ein Ventil mit der Verteilerleitung verbunden ist und Funktionselemente aufweist, die es ermöglichen, die Anschlussleitung in die Desinfektion des Dialysegerätes einzubeziehen. Zur Vorbereitung einer chemischen Desinfektion des Dialysegerätes wird der Füllstand in der Flusskammer abgesenkt, vorzugsweise durch Auslassen von Flüssigkeit durch eine vom unteren Teil der Flusskammer abzweigende Leitung in einen Abfluss oder durch die zuführende Anschlussleitung. Durch das Absenken des Füllstandes in der Flusskammer entsteht ein Zwischenraum zwischen dem Wasserspiegel in der Flusskammer und dem oberen Anschlussbereich an die Verteilerleitung, wobei der freie Einlauf sicher stellt, dass keinerlei Desinfektionsflüssigkeit in die Verteilerleitung gelangen kann. Außerdem kann vorgesehen sein, dass die Flusskammer die Funktion des bisher in Dialysegeräten üblicherweise vorgesehenen Vorlaufgefäßes übernimmt, so dass ein gesondertes Vorlaufgefäß entfallen kann.

Weitere Einzelheiten der Erfindung ergeben sich aus der nachfolgenden Beschreibung von Ausführungsbeispielen und aus den Zeichnungen. Dabei zeigen:
- Figur 1: eine Anordnung entsprechend der Erfindung in rein schematischer Darstellung,
- Figur 2: ein Beispiel mit einigen Gestaltungsdetails für den Anschluss der in der Ankopplungsvorrichtung eingesetzten Flusskammer an die Verteilerleitung.

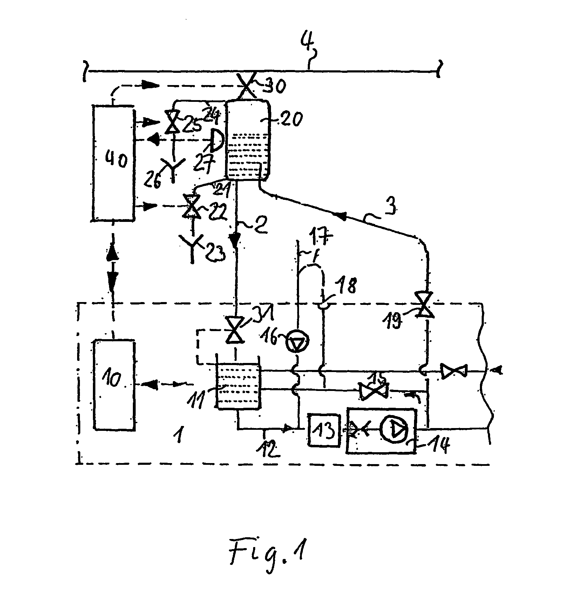

Die Darstellung des Dialysegeräts 1 in Figur 1 beschränkt sich im wesentlichen auf Funktionselemente, die in Verbindung mit dem Erfindungsgegenstand von Bedeutung sind. Das skizzierte Schema ist nur als Beispiel zu betrachten und kann in verschiedener Weise abgewandelt werden, ohne die Anwendbarkeit der Erfindung zu beeinträchtigen. Das von der zentralen Versorgungseinrichtung gelieferte und über die Leitung 2 zugeführte Wasser gelangt in ein Zulaufgefäß 11, das in einen Flüssigkeitskreislauf mit der Heizvorrichtung 13, der Entgasungsvorrichtung 14 und dem Ventil 15 einbezogen ist. Dieser Kreislauf dient u.a. der Erwärmung auf die Anwendungstemperatur und der Abscheidung überschüssig gelöster Luft. Während des Dialysebetriebes wird diesem Kreislauf außerdem aus einem externen Vorratsbehälter mittels einer Dosierpumpe 16 über die Ansaugleitung 17 das zur Bildung der Dialysierlösung erforderliche Dialysekonzentrat zugeführt.

Es wird betont, dass das Zulaufgefäß 11 nicht unbedingt erforderlich ist und bei einer erfindungsgemäßen Anordnung entfallen kann.

Bei einer chemischen Desinfektion gelangt auf dem gleichen Wege, über die Leitung 17 und die Dosierpumpe 16, statt des Dialysekonzentrats das Desinfektionsmittel-Konzentrat in den Kreislauf des Dialysegeräts. Vor Beginn des anschließenden Spülvorgangs muss die Saugleitung 17 mit dem Spülanschluss 18 verbunden werden, um auch das Ansaugsystem in die Spülung einzubeziehen.

Die Anwendung der Erfindung sieht vor, dass das Dialysegerät neben der zuführenden Anschlussleitung2 eine zweite, rückführende Anschlussleitung 3 aufweist. Am Ausgang der Entgasungsvorrichtung 14 steht ein positiver Druck zur Verfügung, dessen Höhe vom Strömungswiderstand des Ventils 15 (evtl. mit einer zusätzlich eingefügten Drossel) abhängt. Bei dem in Figur. 1 gezeigten Beispiel ist die abführende Anschlussleitung vom Ausgang der Entgasungsvorrichtung abgezweigt und über das Ventil 19 nach außen geführt. Durch zeitweiliges Schließen des Ventils 15 kann der über das Ventil 19 nach außen geleitete Fluss bei Bedarf erhöht werden.

Wesentlicher Bestandteil der Vorrichtung entsprechend der Erfindung ist die Flusskammer 20, deren oberer Teil über das Ventil 30 an die Versorgungsleitung 4 des zentralen Wasseraufbereitungssystems angeschlossen wird. Die zuführende Anschlussleitung 2 und die rückführende Anschlussleitung 3 des Dialysegeräts gehen vom unteren Teil der Flusskammer ab bzw. münden dort ein. Vom unteren Teil der Flusskammer 20 zweigt außerdem die Leitung 21 mit dem Ventil 22 ab. Diese Leitung mündet unterhalb der Flusskammer offen in einen Abfluss 23. Eine weitere Leitung 24 mit dem Ventil 25, die vom oberen Teil der Flusskammer abzweigt, mündet offen in den Abfluss 26, der über dem Abfluss 23 liegt. Zur Überwachung des Füllstandes in der Flusskammer ist mindestens ein Füllstandssensor 27 vorgesehen. Die Steuerungseinheit 40 dient der Ansteuerung der Ventile und Verarbeitung der Sensorsignale. Sie korrespondiert mit der Steuerungseinheit 10 des Dialysegeräts, um den weiter unten beschriebenen Funktionsablauf herzustellen. Das Dialysegerät ist in Figur 1 durch das gestrichelte Rechteck angedeutet.

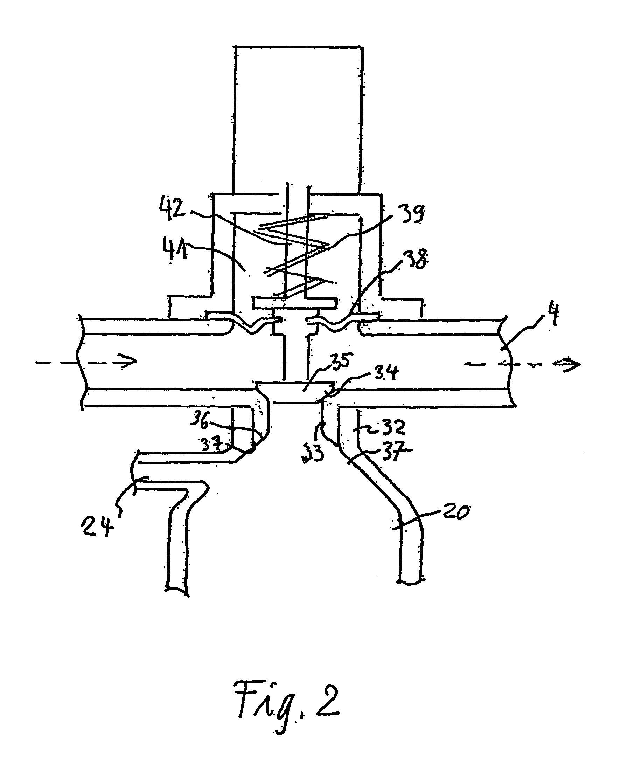

Figur 2 zeigt die unmittelbare Ankoppelung der Flusskammer 20 an die Verteilerleitung 4. Von der Verteilerleitung 4 zweigt seitlich ein Rohrstutzen 33 ab, der von einem zylindrischen Kragen oder Halsabschnitt 32 am oberen Ende des Flusskammer 20 dicht umschlossen ist. Der Halsabschnitt 32 ist auf geeignete Weise an dem Rohrstutzen 22 befestigt. Der Rohrstutzen 33 hat eine kegelstumpfförmige Randfläche 36, die glatt in einen kegelförmigen Innenabschnitt 37 der Flusskammer 20 übergeht, so dass hier die Ausbildung eines Totraums vermieden ist, der günstige Vermehrungsbedingungen für Mikroorganismen mit sich bringen könnte.

Dicht unterhalb des Verbindungsbereichs des Halsabschnitts 32 mit dem Rohrstutzen 33 zweigt die oben erwähnte Leitung 24 ab.

Der Rand der Abzweigung des Rohrstutzens 33 ist kegelstumpfförmig zu einem Ventilsitz 34 abgeschrägt, an dem ein entsprechend geformter im wesentlichen tellerförmiger Ventilkörper 35 dicht anliegt. Der Ventilkörper 35 ist beispielsweise durch eine elektrische Betätigungsvorrichtung in der Darstellung der Figur 2 nach oben bewegbar, um den Zulauf von Wasser in die Flusskammer 20 frei zu geben. In einer von einer Gummidichtung 38 abgedichteten obere Kammer 41 sitzt auf einem Ventilschaft 42 eine Druckfeder, die den Ventilkörper 35 wieder in Anlage an den Ventilsitz 34 bringen kann.

Zur Vorbereitung einer chemischen Desinfektion des Dialysegeräts 1 wird bei geschlossenem Ventil 30 zunächst der Füllstand in der Flusskammer 20 auf ein mittleres Niveau (entsprechend der Position des Füllstandssensors 27) abgesenkt. Hierzu werden die Ventile 22 und 25 geöffnet, so dass Wasser über die Leitung 21 ausfließt und Luft über die Leitung 24 in die Flusskammer nachströmt. Danach wird das Ventil 22 geschlossen, das Ventil 25 bleibt geöffnet.

Nach Anschließen des Desinfektionsmittelkonzentrats an die Leitung 17 kann das Dialysegerät im Desinfektionsmodus betrieben werden. Dabei fließt Desinfektionslösung durch die Anschlussleitungen 2 und 3 des Dialysegeräts und durch die Flusskammer 20. Bei Bedarf kann durch zeitweilige Erhöhung des Flusses in der rückführenden Anschlussleitung 3 erreicht werden, dass die gesamte Innenfläche der Flusskammer mit Desinfektionslösung besprüht wird. Eine während des Desinfektionsvorgangs evtl. notwendige Absenkung des Füllstandes in der Flusskammer ist jederzeit durch Öffnen des Ventils 22 möglich.

Zum Zwecke der Spülung des Dialysegeräts, die im Anschluss an die Desinfektion notwendig ist, wird die Wasserzufuhr aus der Verteilerleitung 4 des zentralen Versorgungssystem durch Öffnen des Ventils 30 freigegeben. In den Ablauf des Spülprogramms des Dialysegeräts wird eine vollständige Ausspülung der Flusskammer 20 mit allen angeschlossenen Leitungen einbezogen, indem auch die Ventile 22 und 25 zeitweise abwechseln geöffnet werden.

## Patentansprüche

1. Anordnung zur Wasserversorgung eines Dialysegerätes, mit einer Verteilerleitung einer zentralen Wasseraufbereitungseinrichtung und einem Dialysegerät, das an die Verteilerleitung angekoppelt ist,
**gekennzeichnet durch**
eine Flusskammer (20), die außerhalb des Dialysegerätes angeordnet ist und mit ihrem oberen Teil über ein Schaltventil (30) mit der Verteilerleitung (4) verbunden ist,
wobei die Flusskammer (20) über eine zuführende Anschlussleitung (2) mittels eines im unteren Bereich der Flusskammer (20) befindlichen Anschlusses und über eine rückführende Anschlussleitung (3) mit dem Dialysegerät verbunden ist,
wobei die Flusskammer ferner eine vom oberen Bereich der Flusskammer (20) abzweigenden Leitung (24), die über ein Ventil (25) zu einem Abfluss (26) führt, und wenigstens einen Füllstandssensor (27) zur Messung/Überwachung des Füllstandes aufweist.

2. Anordnung nach Anspruch 1,
**dadurch gekennzeichnet,**
**dass** die Flusskammer (20) eine vom unteren Bereich abzweigende Leitung (21) aufweist, die über ein Ventil (22) zu einem Abfluss (23) führt.

3. Anordnung nach Anspruch 1,
**dadurch gekennzeichnet,**
**dass** der Anschluss der Flusskammer (20) für die rückführende Anschlussleitung (3) ebenfalls im unteren Bereich der Flusskammer (20) angeordnet ist.

4. Anordnung nach Anspruch 1,
**dadurch gekennzeichnet,**
**dass** der wenigstens eine Füllstandssensor (27) etwa auf halber Höhe der Flusskammer (20) angeordnet ist.

5. Anordnung nach einem der Ansprüche 1 bis 4,
**dadurch gekennzeichnet,**
**dass** die rückführende Anschlussleitung (3) vom Ausgang einer Entgasungsvorrichtung (14) des Dialysegerätes abzweigt.

6. Anordnung nach einem der Ansprüche 1 bis 5,
**dadurch gekennzeichnet,**
**dass** die rückführende Anschlussleitung (3) über ein Ventil (19) aus dem Dialysegerät heraus führt.

7. Anordnung nach einem der Ansprüche 1 bis 6,
**dadurch gekennzeichnet,**
**dass** die zuführende Anschlussleitung (2) über ein Ventil (31) zu einem Zulaufgefäß (11) des Dialysegerätes führt.

8. Anordnung nach einem der Ansprüche 1 bis 7,
**dadurch gekennzeichnet,**
**dass** die Flusskammer (20) direkt an der Verteilerleitung (4) angesetzt ist.

9. Anordnung nach Anspruch 8,
**dadurch gekennzeichnet,**
**dass** die Flusskammer (20) einen oberen Halsabschnitt (32) hat, der einen seitlichen Rohrstutzen (33) der Verteilerleitung (4) dicht umgreift.

10. Anordnung nach Anspruch 9,
**dadurch gekennzeichnet,**
**dass** am Rand des seitlichen Rohrstutzens (33) ein Ventilsitz (34) für einen quer zur Längsrichtung der Verteilerleitung (4) bewegbaren Ventilkörper (35) ausgebildet ist.

## Claims

1. Arrangement for supplying water to a dialysis machine, having a distributor line of a central water preparation device and a dialysis machine which is coupled to the distributor line,
**characterised by**
a flow chamber (20) which is arranged outside the dialysis machine and is joined by its upper part to the distributor line (4) via a switching valve (30),
the flow chamber (20) being connected to the dialysis machine via a supply connection line (2) by means of a connection located in the lower region of the flow chamber (20) and via a return connection line (3),
the flow chamber also having a line (24) branching off from the upper region of the flow chamber (20), which line leads via a valve (25) to an outflow (26), and at least one level sensor (27) for measurement/monitoring of the level.

2. Arrangement according to claim 1,
**characterised in that**
the flow chamber (20) has a line (21) branching off from the lower region, which line leads via a valve (22) to an outflow (23).

3. Arrangement according to claim 1,
**characterised in that**
the connection of the flow chamber (20) for the return connection line (3) is likewise arranged in the lower region of the flow chamber (20).

4. Arrangement according to claim 1,
**characterised in that**
the at least one level sensor (27) is arranged at approximately half the height of the flow chamber (20).

5. Arrangement according to any one of claims 1 to 4,
**characterised in that**
the return connection line (3) branches off from the outlet of a degassing device (14) of the dialysis machine.

6. Arrangement according to any one of claims 1 to 5,
**characterised in that**
the return connection line (3) leads out of the dialysis machine via a valve (19).

7. Arrangement according to any one of claims 1 to 6,
**characterised in that**
the supply connection line (2) leads via a valve (31) to an inflow container (11) of the dialysis machine.

8. Arrangement according to any one of claims 1 to 7,
**characterised in that**
the flow chamber (20) is joined directly to the distributor line (4).

9. Arrangement according to claim 8,
**characterised in that**
the flow chamber (20) has an upper neck portion (32) which engages sealingly around a lateral tubular connector (33) of the distributor line (4).

10. Arrangement according to claim 9,
**characterised in that**
at the rim of the lateral tubular connector (33) there is formed a valve seat (34) for a valve body (35), which valve body is movable transversely with respect to the longitudinal direction of the distributor line (4).

## Revendications

1. Dispositif d'alimentation en eau d'un appareil de dialyse, avec une conduite de distribution d'un dispositif central de traitement de l'eau et un appareil de dialyse qui est accouplé à la conduite de distribution,
**caractérisé par**
une chambre de flux (20) qui est agencée hors de l'appareil de dialyse et est reliée avec sa partie supérieure à la conduite de distribution (4) par le biais d'une soupape de commande (30),
dans lequel la chambre de flux (20) est reliée à l'appareil de dialyse par le biais d'une conduite de raccordement d'amenée (2) au moyen d'un raccord situé dans la zone inférieure de la chambre de flux (20) et par le biais d'une conduite de raccordement de retour (3),
dans lequel la chambre de flux présente en outre une conduite (24) bifurquant de la zone supérieure de la chambre de flux (20) et menant à une évacuation (26) par le biais d'une soupape (25), et au moins un capteur de niveau de remplissage (27) pour mesurer / surveiller le niveau de remplissage.

2. Dispositif selon la revendication 1,
**caractérisé en ce que**
la chambre de flux (20) présente une conduite (21) bifurquant de la zone inférieure et menant à une évacuation (23) par le biais d'une soupape (22).

3. Dispositif selon la revendication 1,
**caractérisé en ce que**
le raccord de la chambre de flux (20) pour la conduite de raccordement de retour (3) est également disposé dans la zone inférieure de la chambre de flux (20).

4. Dispositif selon la revendication 1,
**caractérisé en ce que**
l'au moins un capteur de niveau de remplissage (27) est disposé à peu près à mi-hauteur de la chambre de flux (20).

5. Dispositif selon l'une quelconque des revendications 1 à 4,
**caractérisé en ce que**
la conduite de raccordement de retour (3) bifurque de la sortie d'un dispositif de dégazage (14) de l'appareil de dialyse.

6. Dispositif selon l'une quelconque des revendications 1 à 5,
**caractérisé en ce que**
la conduite de raccordement de retour (3) mène hors de l'appareil de dialyse par le biais d'une soupape (19).

7. Dispositif selon l'une quelconque des revendications 1 à 6,
**caractérisé en ce que**
la conduite de raccordement d'amenée (2) mène à un récipient d'alimentation (11) de l'appareil de dialyse par le biais d'une soupape (31).

8. Dispositif selon l'une quelconque des revendications 1 à 7,
**caractérisé en ce que**
la chambre de flux (20) est mise en place directement au niveau de la conduite de distribution (4).

9. Dispositif selon la revendication 8,
**caractérisé en ce que**
la chambre de flux (20) a une section de col supérieure (32) qui entoure de manière étanche un raccord tubulaire latéral (33) de la conduite de distribution (4).

10. Dispositif selon la revendication 9,
**caractérisé en ce que**
un siège de soupape (34) pour un corps de soupape (35) mobile transversalement au sens longitudinal de la conduite de distribution (4) est réalisé au bord du raccord tubulaire latéral (33).
